(19) Europäisches Patentamt
European Patent Office
Office européen des brevets

(11) **EP 3 389 439 B1**

(12) **EUROPÄISCHE PATENTSCHRIFT**

(45) Veröffentlichungstag und Bekanntmachung des Hinweises auf die Patenterteilung:
**07.10.2020 Patentblatt 2020/41**

(21) Anmeldenummer: **16816261.8**

(22) Anmeldetag: **16.12.2016**

(51) Int Cl.:
*A45D 44/00* (2006.01)   *G16C 20/30* (2019.01)
*G06Q 30/02* (2012.01)   *G06Q 30/06* (2012.01)
*A45C 5/02* (2006.01)

(86) Internationale Anmeldenummer:
**PCT/EP2016/081366**

(87) Internationale Veröffentlichungsnummer:
**WO 2017/103056 (22.06.2017 Gazette 2017/25)**

(54) **VERFAHREN UND DATENVERARBEITUNGSVORRICHTUNG ZUM COMPUTERGESTÜTZTEN ERMITTELN EINES HAARFÄRBEMITTELS ZUM FÄRBEN VON HAAREN IN EINER WUNSCHHAARFARBE UND VORRICHTUNG ZUM HERSTELLEN EINES INDIVIDUELL ERMITTELTEN HAARFÄRBEMITTELS**

METHOD AND DATA PROCESSING DEVICE FOR THE COMPUTER-ASSISTED DETERMINATION OF A HAIR DYEING AGENT FOR DYEING HAIR IN A DESIRED HAIR COLOR AND DEVICE FOR PRODUCING AN INDIVIDUALLY DETERMINED HAIR DYEING AGENT

PROCÉDÉ ET DISPOSITIF DE TRAITEMENT DE DONNÉES PERMETTANT LA DÉTERMINATION ASSISTÉE PAR ORDINATEUR D'UNE TEINTURE CAPILLAIRE POUR COLORER DES CHEVEUX DANS UNE COLORATION DE CHEVEUX SOUHAITÉE ET DISPOSITIF DE FABRICATION D'UNE TEINTURE CAPILLAIRE DÉTERMINÉE INDIVIDUELLEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.12.2015 DE 102015225459**

(43) Veröffentlichungstag der Anmeldung:
**24.10.2018 Patentblatt 2018/43**

(73) Patentinhaber: **Henkel AG & Co. KGaA**
**40589 Düsseldorf (DE)**

(72) Erfinder: **KNÜBEL, Georg**
**40219 Düsseldorf (DE)**

(56) Entgegenhaltungen:
**EP-A1- 1 374 720    WO-A1-2006/090363**
**WO-A1-2011/024160    WO-A2-01/87245**
**WO-A2-2012/127429    US-A1- 2004 000 015**
**US-A1- 2006 033 907**

**Beschreibung**

Die Erfindung betrifft ein Verfahren und eine Datenverarbeitungsvorrichtung zum computergestützten Ermitteln eines Haarfärbemittels zum Färben von Haaren in einer Wunschhaarfarbe und eine Vorrichtung zum Herstellen eines individuell ermittelten Haarfärbemittels.

Bei herkömmlichen Verfahren zum Ermitteln eines Haarfärbemittels zum Färben von Haaren in einer Wunschhaarfarbe stehen insbesondere dem Endkunden zum Definieren eines Ausgangszustands der Haare, die gefärbt werden sollen, beispielsweise einer Grundhaarfarbe, eines Ergrauungsgrads oder eines Schädigungsgrads, typischerweise nur wenige Werte zur Auswahl, beispielsweise drei Werte für die Grundhaarfarbe (z.B. blond, mittelblond und braun), drei Werte für den Ergrauungsgrad (0%, 50% und 100%) und drei Werte für den Schädigungsgrad (keine Schädigung, leichte Schädigung, starke Schädigung).

Dies erlaubt Ermitteln eines erwarteten Färbeergebnisses allerdings höchstens für einen Fall, bei welchem ein Ausgangszustand der Haare einer Person zufälligerweise gerade genau einer verfügbaren Kombination von zum Auswählen angebotenen Werten zum Definieren eines Ausgangszustands (beispielsweise blond, 50% und leichte Schädigung) entspricht.

Für eine Person, die ihre Haare färben (lassen) möchte, ist ein Übereinstimmen von erwartetem und erzieltem Färbeergebnis jedoch von hoher Bedeutung.

Dokument US 2006/033907 A1 beschreibt ein Verfahren zur Bestimmung einer Haarfärbeformel, um eine Zielfarbe zu erreichen, indem auf Informationen aus einer Datenbank zugegriffen wird. Dokument WO 2006/090363 A1 beschreibt ein System und ein Verfahren zur Bestimmung einer Haarfärbebehandlung auf der Grundlage von Berechnungen, die an einem Spektrum von Haaren durchgeführt werden. Dokument WO 2011/024160 A1 beschreibt ein Verfahren zur Bestimmung und Herstellung einer Haarfärbeformulierung. Dokument EP 1374720 A1 beschreibt ein Verfahren und eine Vorrichtung zur Vorhersage des Ergebnisses einer Haarfärbebehandlung. Dokument WO 2012/127429 A2 beschreibt ein Verfahren zur Herstellung eines Haarfärbeproduktes, das den Bedürfnissen des Benutzers entspricht. Dokument US 2004/000015 A1 beschreibt ein Verfahren zur Änderung der Haarfarbe auf der Grundlage eines gemessenen Reflexionsspektrums und der Berechnung einer Haarbehandlung. Dokument WO 01/87245 A2 beschreibt eine Methode und ein System zur Analyse der Haare, zur Vorhersage der erreichbaren Haarfarben und zur Empfehlung von Haarfärbemitteln.

Die Erfindung bezieht sich auf ein Verfahren und eine Vorrichtung, wie im beigefügten Anspruchssatz beschrieben.

In verschiedenen Ausführungsbeispielen wird ein Verfahren bereitgestellt, welches einen individuellen Ausgangszustand der Haare berücksichtigen kann bei einem Ermitteln eines Haarfärbemittels zum Färben von Haaren in einer Wunschhaarfarbe. Dabei kann der Ausgangszustand der Haare beispielsweise mittels Messungen ermittelt werden.

In verschiedenen Ausführungsbeispielen können beim Ermitteln des Haarfärbemittels zum Färben von Haaren in einer Wunschhaarfarbe ferner Wunschwerte für weitere Eigenschaften von Haarfarben berücksichtigt werden.

Allgemein können Eigenschaften von Haarfarben beispielsweise eine Haarfarbinformation (eine Farbe), eine Waschechtheit, eine Lichtechtheit, eine Grauabdeckung oder weitere Eigenschaften aufweisen. Dabei können die Haarfarben mittels Färbens von Haar mit einem Haarfärbemittel, auch als Färbevorgang bezeichnet, erzeugt sein.

Unter einer "Farbe" kann hierin ein Zusammenwirken eines Farbtons (d.h. eines spektralen Farbeindrucks, auch als Buntton bezeichnet, was als das verstanden werden kann, was als die "eigentliche Farbe" angesehen wird), einer Farbintensität (d.h. wie intensiv die Farbe erscheint, z.B. verglichen mit einem neutralen Grau, was auch als Sättigung, Farbsättigung, Buntheit, Chromatizität, Chromazität oder Farbtiefe bezeichnet wird) und einer Helligkeit (d.h. wie hell oder dunkel die Farbe erscheint) verstanden werden.

In verschiedenen Ausführungsbeispielen kann die Farbinformation beispielsweise eine Parametrisierung in einem bekannten Farbraum aufweisen, beispielsweise in einem L*a*b*-Farbraum (wobei L* die Helligkeit einer Farbe angibt, a* den Grün- und Rotanteil und b* den Blau- und Gelbanteil der Farbe; mitunter wird hierin auch als verkürzende Schreibweise Lab bzw. einzeln L, a, bzw. b verwendet) in einem RGB-Farbraum durch Farbanteile in Rot, Grün und Blau, in einem CMYK-Farbraum durch Farbanteile in Cyan, Magenta, Gelb und Schwarz, oder in einem beliebigen anderen Farbraum.

Unter dem Begriff "Farbton" kann hierin, wie oben beschrieben, der spektrale Farbeindruck einer Farbe verstanden werden, unabhängig davon, wie dieser parametrisiert sein kann, beispielsweise als ein Punkt in einem zweidimensionalen Farbraum (z.B. a*b* des L*a*b*-Systems) oder ein Verhältnis von Farbanteilen (wie z.B. beim RGB-Farbraum oder beim CMYK-Farbraum).

In verschiedenen Ausführungsbeispielen kann ein Farbraum, dem die Farbinformation (z.B. die Haarfarbinformation des gefärbten Haars oder des Haars vor der Färbung, was auch als Grundhaarfarbe bezeichnet wird) entstammt, oder in welchem die Farbinformation dargestellt wird (beispielsweise wenn eine Haarfarbe dargestellt wird, siehe unten) so beschaffen sein, dass eine ermittelte oder dargestellte Farbe unabhängig von einem Medium ist, durch welches die Farbe ermittelt oder dargestellt wird (z.B. Farbmessgerät, Bildschirm, Drucker, Scanner, menschliches Auge, usw.). Der Farbraum kann beispielsweise ein L*a*b*-Farbraum sein, die Farbinformation ein beispielsweise mittels a* und b* parametrisierter Farbton. Die einheitliche Darstellung in dem mediumunabhängigen Farbraum kann es beispielsweise ermöglichen, ein realitätsnahes zu erwartendes Färbeergebnis zu präsentieren, beispielsweise indem eine mittels Färbens erzielte Farbe beim Betrachter des gefärbten Haars denselben Farbeindruck hinterlässt wie in einer Darstellung des zu erwartenden Ergebnisses, beispielsweise als ein Verpackungsaufdruck, eine Anzeige an einem Computerbildschirm, o.ä.

Haarfärbemittel können eine Mischung aus verschiedenen Farbstoffvorstufen und/oder Farbstoffen aufweisen und können deshalb auch als Färbemischung bezeichnet werden.

Eine Vorhersage der genannten Eigenschaften von Haarfarben, also ein Ermitteln eines zu erwartenden Färbeergebnisses, ohne den Färbevorgang tatsächlich ausgeführt zu haben, kann beim Haarefärben, also zum Erzeugen einer Haarfarbe, schwieriger sein als in verwandten Gebieten der Farberzeugung, z.B. beim Fotodruck, weil beim Haarefärben typischerweise keine Farbstoffe eingesetzt werden, zumindest nicht unmittelbar, sondern Farbstoffvorstufen. Während eines Färbevorgangs kann sich dann eine Vielzahl von verschiedenen Farbstoffen bilden, deren Eigenschaften als Reinsubstanzen möglicherweise nicht bekannt sind.

Zum Ermitteln von zu erwartenden Haarfarben für einen (z.B. beliebigen) Ausgangszustand und für eine große Zahl von Kombinationen von jeweiligen Konzentrationen einer Mehrzahl von Farbstoffvorstufen können Methoden aus dem Feld der prädiktiven Analytik, für welche üblicherweise der entsprechende englische Begriff "Predictive Analytics" verwendet wird (auch bekannt als "Big Data" (sinngemäß übersetzt "große Datenmenge"), "Data Mining" (übersetzt "Auswertung großer Datenmengen") oder "Machine Learning" (übersetzt "Maschinenlernen")), genutzt werden, um trotz der vielen Parameter präzise Berechnungen von Eigenschaften der Haarfarben, beispielsweise einer Farbinformation, aber auch beispielsweise einer Waschechtheit, einer Grauabdeckung oder/und einer Lichtechtheit, zu ermöglichen.

Der (z.B. beliebige) Ausgangszustand kann beispielsweise einen beliebigen Wert für einen Ausgangszustandsparameter oder eine beliebige Kombination von Werten für eine Mehrzahl von Ausgangszustandsparametern, beispielsweise für eine kontinuierliche Verteilung von Werten für den mindestens einen Ausgangszustandsparameter, aufweisen.

Die große Zahl von Kombinationen von Konzentrationen der Vielzahl von Farbstoffvorstufen kann beispielsweise $10^5$ Kombinationen oder mehr aufweisen, oder beispielsweise eine kontinuierliche Verteilung von Werten für die Konzentrationen der Mehrzahl von Farbstoffvorstufen.

Mittels der Prädiktiven Analytik kann unter Verwendung von Haarfarben-Daten eine Beziehung ermittelt werden zwischen einer Mehrzahl von Färbevoraussetzungsparameter und mindestens einem Färbeergebnisparameter. Dabei kann die Mehrzahl von Färbevoraussetzungsparametern eine Mehrzahl von Konzentrationen von Farbstoffvorstufen und mindestens einen Ausgangszustandsparameter aufweisen.

Mittels den Methoden aus der Prädiktiven Analytik ist es nun möglich, einem Benutzer (z.B. einem Verbraucher) ein Farbresultat zu garantieren, welches so nah wie möglich an seiner Wunschhaarfarbe ist (soweit dies chemisch möglich ist).

In verschiedenen Ausführungsbeispielen kann ein Verfahren zum computergestützten Ermitteln eines Haarfärbemittels zum Färben von Haaren in einer Wunschhaarfarbe bereitgestellt werden.

In verschiedenen Ausführungsbeispielen kann ein Wert für mindestens einen Ausgangszustandsparameter, welcher einen Ausgangszustand der zu färbenden Haare beschreibt, ermittelt werden. Beispielsweise kann jeweils ein Wert für eine Mehrzahl von Ausgangszustandsparametern ermittelt werden. Der mindestens eine Ausgangszustandsparameter kann beispielsweise mindestens eine Ausgangshaarfarbe der zu färbenden Haare aufweisen, wobei die Ausgangshaarfarbe in einem Farbraum, beispielsweise einem L*a*b*-Farbraum, einem RGB-Farbraum, o.ä., parametrisiert sein kann.

In verschiedenen Ausführungsbeispielen kann die mindestens eine Ausgangshaarfarbe eine Mehrzahl von Ausgangshaarfarben aufweisen. Die zu färbenden Haare können beispielsweise eine Mehrzahl von Ausgangshaarfarben aufweisen. Beispielsweise kann eine erste Ausgangshaarfarbe an Spitzen der Haare verschieden sein von einer zweiten Ausgangshaarfarbe an einem Haaransatz der Haare und/oder von einer dritten Ausgangshaarfarbe an einem Hauptteil der Haare, und/oder von einer vierten Ausgangshaarfarbe an Strähnen der Haare, o.ä.

Der mindestens eine Ausgangszustandsparameter kann zusätzlich zur Ausgangshaarfarbe einen Ergrauungsgrad der Haare, einen Schädigungsgrad der Haare o.ä. aufweisen.

In verschiedenen Ausführungsbeispielen kann eine Ausgangshaarfarbe von einer Mehrzahl von Ausgangshaarfarben ausgewählt werden, beispielsweise durch eine Person, deren Haare es sind, als eine Ausgangshaarfarbe, welche am repräsentativsten für die Haare ist, beispielsweise eine Ausgangshaarfarbe, welche für einen Farbeindruck, welchen die Haare bei einem Betrachter hervorrufen, am prägendsten ist.

In verschiedenen Ausführungsbeispielen kann eine Mehrzahl von mittels Färbens mit jeweils einem verfügbaren Haarfärbemittel von einer Mehrzahl von verfügbaren Haarfärbemitteln erzielbaren Haarfarben ermittelt werden.

Dem Ermitteln der erzielbaren Haarfarben können in verschiedenen Ausführungsbeispielen eine Beziehung zwischen einer Mehrzahl von Färbevoraussetzungsparametern und erzielten Haarfarben, welche basierend auf einer anhand von Haarfarben-Daten für eine Mehrzahl von Färbevorgängen ermittelt wurde oder wird, und der mindestens eine Wert für den mindestens einen Ausgangszustandsparameter zugrunde gelegt werden.

In verschiedenen Ausführungsbeispielen kann beim Ermitteln der erzielbaren Haarfarben mindestens ein Wunschwert für einen zusätzlichen (z.B. zusätzlich zur Haarfarbe) Färbeergebnisparameter berücksichtigt werden. Beispielsweise können die erzielbaren Haarfarben beim Ermitteln der erzielbaren Haarfarben auf diejenigen beschränkt werden, die auch den (z.B. Mindest-) Wert für den mindestens einen zusätzlichen Färbeergebnisparameter, beispielsweise eine geforderte (Mindest-)Waschechtheit oder eine geforderte (Mindest-)Grauabdeckung aufweist.

In verschiedenen Ausführungsbeispielen kann die Mehrzahl von verfügbaren Haarfärbemitteln eine Mehrzahl von vorkonfektionierten Haarfärbemitteln aufweisen, beispielsweise Haarfärbemittel, welche im Handel erhältlich sind. In verschiedenen Ausführungsbeispielen kann die Mehrzahl von verfügbaren Haarfärbemitteln eine Mehrzahl von Haarfärbemitteln aufweisen, welche jeweils eine zuvor noch nicht bekannte, nicht verwendete oder nicht ausgefärbte Kombination von Konzentrationen von Farbstoffvorstufen aufweisen. In verschiedenen Ausführungsbeispielen kann die Mehrzahl von verfügbaren Haarfärbemitteln sowohl vorkonfektionierte als auch unbekannte Haarfärbemittel aufweisen.

In verschiedenen Ausführungsbeispielen kann das Verfahren mittels einer Datenverarbeitungsvorrichtung ausgeführt werden.

Die Datenverarbeitungsvorrichtung kann beispielsweise einen Computer aufweisen, oder jede andere Datenverarbeitungsvorrichtung, die geeignet ist, die Daten zu speichern und bereitzustellen und das Prädiktive-Analytik-Verfahren auszuführen, also beispielsweise jede Datenverarbeitungsvorrichtung mit hinreichend großem Datenspeicher und hinreichend leistungsfähigem Prozessor.

Anschaulich beschrieben kann die Prädiktive Analytik genutzt werden, um anhand von Haarfarben-Daten, welche Ergebnisse von ggf. einer großen Zahl von Testfärbungen aufweisen können, eine Beziehung zu ermitteln zwischen einer Mehrzahl von Färbevoraussetzungsparametern (welche ein Färbeergebnis beeinflussen können) und mindestens einem Färbeergebnisparameter (welcher ein Färbeergebnis beschreibt). Die ermittelte Beziehung, auch als Modell bezeichnet, kann genutzt werden, um auch für nicht in den Haarfarben-Daten vorhandene Kombinationen von Färbevoraussetzungsparametern, beispielsweise für neue Färbemittel, d.h. Färbemittel mit einer neuen Kombination von Konzentrationen von Farbstoffvorstufen, mindestens eine Eigenschaft einer erzielten Haarfarbe, z.B. eine Farbe, eine Grauabdeckung, eine Waschechtheit und/oder eine Lichtechtheit, zu ermitteln. Die Mehrzahl von erzielten Haarfarben kann eine Gesamtheit oder einen Teil einer Gesamtheit von Haarfarben bilden, welche für eine beliebige Kombination von Färbevoraussetzungsparametern theoretisch erzielbar sind. Der mindestens eine Wert für den mindestens einen Ausgangszustandsparameter kann genutzt werden, um aus der Gesamtheit von theoretisch erzielbaren Haarfarben diejenigen auszuwählen, welche angesichts des mindestens einen Werts für den Ausgangsparameter noch erreichbar sind.

Beispielsweise können bei einer dunkelbraunen Ausgangshaarfarbe (sofern die Haare nicht vor dem Färben aufgehellt werden) aus der Gesamtheit der theoretisch erzielbaren Haarfarben nur diejenigen Haarfarben erzielbare Haarfarben sein, die mindestens genauso dunkel sind wie die dunkelbraune Ausgangshaarfarbe.

Aus den erzielbaren Haarfarben, die mittels eines jeweiligen Färbevorgangs mit einem Haarfärbemittel erzielt werden können, kann in verschiedenen Ausführungsbeispielen eine Wunschhaarfarbe ausgewählt werden.

Das Auswählen der Wunschhaarfarbe kann in verschiedenen Ausführungsbeispielen beispielsweise ein Auswählen nach einem Präsentieren der erzielbaren Haarfarben erfolgen. Die erzielbaren Haarfarben können beispielsweise mittels einer Anzeigevorrichtung, z.B. mittels eines Bildschirms, z.B. eines Computerbildschirms, oder mittels einer sonstigen Ausgabevorrichtung präsentiert werden, beispielsweise ausgedruckt werden. Beim Präsentieren können beispielsweise die erzielbaren Haarfarben als Farben präsentiert werden. Dabei können die Haarfarben beispielsweise in einem mediumunabhängigen Farbraum, z.B. im L*a*b*-Farbraum, parametrisiert sein, was eine sehr realitätsnahe Darstellung der erzielbaren Haarfarben ermöglicht, oder die Haarfarben können von einem Farbraum in einen Farbraum, welcher von der Ausgabevorrichtung erwartet wird, transformiert werden. In verschiedenen Ausführungsbeispielen kann darauf verzichtet werden, die Farben selbst wiederzugeben. Stattdessen können beispielsweise Werte für eine jeweilige Parametrisierung im Farbraum angegeben werden.

Das Auswählen kann in verschiedenen Ausführungsbeispielen ein Eingeben der Auswahl in eine Datenverarbeitungsvorrichtung, z.B. einen Computer, aufweisen. Dabei kann der Eingabevorgang jede beliebige Art der Eingabe aufweisen, beispielsweise ein Berühren eines Bildschirms, ein Anklicken eines Bildschirmbereichs mit einem Mauszeiger, ein Eingeben von Informationen mittels Tastatur, eine Sprachanweisung.

In verschiedenen Ausführungsbeispielen kann ein Angeben einer Wunschhaarfarbe vor dem Präsentieren der erzielbaren Haarfarben oder sogar vor dem Ermitteln der erzielbaren Haarfarben erfolgen, beispielsweise anhand ihrer Werte für die Parametrisierung im Farbraum oder einer Präsentation solcher allgemeinen parametrisierten Farben.

In einem solchen Fall kann das Auswählen der Wunschhaarfarbe automatisch erfolgen. Sofern die vorbestimmte Wunschhaarfarbe eine der erzielbaren Haarfarben ist, kann diese dann automatisch als Wunschhaarfarbe ausgewählt werden.

Im Fall, dass die eigentliche Wunschhaarfarbe eine nicht erzielbare Haarfarbe ist, kann das Präsentieren der erzielbaren Haarfarben eine Angabe über den Farbabstand (ΔE) zur eigentlichen Wunschhaarfarbe umfassen.

In verschiedenen Ausführungsbeispielen kann ferner ein Toleranzbereich bereitgestellt werden, wobei der Toleranzbereich ein Farbabstand zur Wunschhaarfarbe sein kann, der toleriert wird. Das Bestimmen des Haarfärbemittels zum Färben von Haaren in der Wunschhaarfarbe kann dann zusätzlich auf dem Toleranzbereich basieren.

Gemäß verschiedenen Ausführungsbeispielen kann der Toleranzbereich kleiner sein als eine menschliche Wahrnehmungsschwelle für den Farbabstand. Die menschliche Wahrnehmungsschwelle kann dabei in einer üblichen Weise definiert sein, beispielsweise als eine Schwelle, bei welcher ein durchschnittlicher Betrachter einen Farbunterschied wahrnimmt, bei welcher ein geschulter/empfindlicher Betrachter einen Farbunterschied wahrnimmt, oder bei welcher ein unempfindlicher Betrachter einen Farbunterschied wahrnimmt.

In verschiedenen Ausführungsbeispielen kann es eine Mehrzahl von Haarfärbemitteln geben, welche beim Färben der Haare zur Wunschhaarfarbe (mit oder ohne definierten Toleranzbereich) führen. In einem solchen Fall kann das Haarfärbemittel willkürlich ausgewählt werden, oder es können weitere Kriterien zum Ermitteln des Haarfärbemittels herangezogen werden, beispielsweise ein Preis für das Haarfärbemittel, eine Wirkung auf die Haare (z.B. ob und ggf. wie stark das Haarfärbemittel die Haare schädigen kann), eine Verfügbarkeit des Haarfärbemittels, usw.

In verschiedenen Ausführungsbeispielen kann unter Verwendung der ausgewählten Wunschhaarfarbe jeweils ein Farbabstand ermittelt werden zwischen einer Mehrzahl von vorkonfektionierten Haarfärbemitteln (welche auch als Retail-Produkte bezeichnet werden können) und der Wunschhaarfarbe.

In verschiedenen Ausführungsbeispielen kann das Haarfärbemittel zum Färben von Haaren in der Wunschhaarfarbe basierend auf der Wunschhaarfarbe und der ermittelten Beziehung zwischen den Färbevoraussetzungsparametern und den erzielten Haarfarben bestimmt werden.

In verschiedenen Ausführungsbeispielen kann als das Haarfärbemittel zum Färben von Haaren in der Wunschhaarfarbe aus den vorkonfektionierten Haarfärbemitteln dasjenige ausgewählt werden, dessen gemäß der ermittelten Beziehung unter Berücksichtigung der Ausgangszustandsparameter einen geringsten Farbabstand zur Wunschhaarfarbe aufweist.

Alternativ kann in verschiedenen Ausführungsbeispielen eine Wichtung des Farbstandes vorgenommen werden. Sollte beispielsweise die exakte Farbrichtung (H, "hue") der Wunschhaarfarbe wichtiger sein als die exakte Helligkeit (L, "lightness"), sollte ΔH stärker in Farbabstand ΔE einfließen als ΔL.

In verschiedenen Ausführungsbeispielen kann als das Haarfärbemittel zum Färben von Haaren in der Wunschhaarfarbe aus den vorkonfektionierten Haarfärbemitteln dasjenige ausgewählt werden, dessen gemäß der ermittelten Beziehung unter Berücksichtigung der Ausgangszustandsparameter eine geringste Farbtonabweichung ΔH, Helligkeitsabweichung ΔL oder Chromaabweichung ΔC zur Wunschhaarfarbe aufweist.

In verschiedenen Ausführungsbeispielen, beispielsweise wenn zusätzlich oder alternativ zu den vorkonfektionierten Haarfärbemitteln auch nicht vorkonfektionierte Haarfärbemittel genutzt werden können, kann, anschaulich ausgedrückt, die Wunschhaarfarbe als Ausgangsparameter in die Beziehung (das Modell) hineingesteckt werden, um ein zugeordnetes Färbemittel, welches beim Färben mit den Färbevoraussetzungsparametern die Wunschhaarfarbe ergibt, zu erhalten, beispielsweise eine Kombination von Färbemittelvorstufen.

Der mindestens eine Färbeergebnisparameter kann in verschiedenen Ausführungsbeispielen ferner weitere Eigenschaften der gefärbten Haarfarbe aufweisen, beispielsweise eine Lichtechtheit, eine Waschechtheit oder eine Fähigkeit zur Grauabdeckung.

In verschiedenen Ausführungsbeispielen kann es sich bei den Messdaten des Datensatzes, d.h. den gemessenen Angaben der Haarfarben-Daten, die Eigenschaften der mittels Färbens erzeugten Haarfarbe beschreiben (z.B. L*, a*, b* für die Farbe, eine Waschechtheit, Lichtechtheit, Grauabdeckung o.ä.), um abhängige Variablen handeln. Mittels eines komplexen mathematischen Modells, welches mittels des Prädiktive-Analytik-Verfahrens gefunden werden kann, kann die Abhängigkeit der abhängigen Variablen von den unabhängigen Variablen (beispielsweise den Konzentrationen der Farbstoffvorstufen, beispielsweise den Konzentrationen, wie sie auf dem Kopf ("on head") vorliegen) modelliert werden. Das heißt, mittels des Prädiktive-Analytik-Verfahrens kann eine Beziehung zwischen den unabhängigen und den abhängigen Variablen (anders ausgedrückt zwischen den Färbevoraussetzungsparametern und den Färbeergebnisparametern) ermittelt werden. Für die Farbe kann dies beispielsweise ausgedrückt werden als:

$$L^*a^*b^* = f(c_1, c_2, c_3, \ldots, c_n),$$

wobei $L^*a^*b^*$ für die Farbparameter steht, $c_n$ ($n=1,\ldots,n$, $n>1$) für Konzentrationen von Farbstoffvorstufen. Dabei kann die Funktion analytisch bekannt sein oder auch nicht. Falls keine analytische Funktion bekannt ist, können die Werte der abhängigen Variablen (der Färbeergebnisparameter) auch mittels numerischer Algorithmen berechnet werden.

Neben Farbstoffvorstufen können auch Farbstoffe in Haarfärbemitteln eingesetzt werden. Entsprechend kann $c_i$ auch für $c_i$ für Konzentrationen von Farbstoffen stehen.

Mögliche unabhängige Variablen können metrisch (kardinal), ordinal oder kategorial sein.

In verschiedenen Ausführungsbeispielen können die unabhängigen Variablen (die Färbevoraussetzungsparameter) Eigenschaften sein, die das Färbeergebnis beeinflussen, beispielsweise die Konzentration einer jeweiligen der Farbstoffvorstufen, die Grundhaarfarbe, ein Schädigungszustand und/oder ein Ergrauungsgrad des Haars, oder Ähnliches.

In verschiedenen Ausführungsbeispielen kann mittels der Prädiktiven Analytik ein Modell erzeugt werden, welches bei vorgegebenen Färbevoraussetzungsparametern (unabhängigen Variablen, Beispiele siehe oben) die Färbeergebnisparameter (abhängige Variablen, Beispiele siehe oben) möglichst genau vorhersagt.

In verschiedenen Ausführungsbeispielen können mittels der Prädiktiven Analytik unabhängige Variablen identifiziert werden, welche keinen oder lediglich einen unbedeutenden Einfluss auf das Modell haben. Anders ausgedrückt kann es sein, dass unabhängige Variablen (Färbevoraussetzungsparameter) in den Haarfarben-Daten vorhanden sind, von welchen angenommen werden konnte, dass sie einen Einfluss auf die abhängigen Variablen (Färbeergebnisparameter) haben, obwohl dies nicht oder lediglich unbedeutend der Fall ist. Diese weniger wichtigen Variablen können mittels des Prädiktive-Analytik-Verfahrens identifiziert werden und ggf. bei nachfolgenden Modellierungen mit vergleichbaren Voraussetzungen außer Betracht gelassen werden.

Bei den einflusslosen unabhängigen Variablen kann es sich in verschiedenen Ausführungsbeispielen um unabhängige Variablen handeln, welche nicht allgemein einen unbedeutenden Einfluss haben, sondern lediglich für einen bestimmten Ausgangszustand oder/und eine bestimmte Wunschhaarfarbe. Als anschauliche Beispiele kann es Farbstoffvorstufen geben, welche z.B. zum Erzeugen eines Rottons nicht oder höchstens in vernachlässigbaren Konzentrationen verwendet werden, oder ein Ergrauungsgrad kann bei einem Haar mit hellblonder Ausgangshaarfarbe für eine erzielte Haarfarbe irrelevant oder wenig relevant sein.

Bei einem Vorliegen von einflusslosen unabhängigen Variablen (Färbevoraussetzungsparametern) kann in verschiedenen Ausführungsbeispielen ein Ermitteln einer Beziehung zwischen der Mehrzahl von Färbevoraussetzungsparametern und mindestens einem Färbeergebnisparameter (z.B. einer Haarfarbe) ohne die einflusslosen Färbevoraussetzungsparameter ausgeführt werden. Prädiktive Analytik kann allgemein als ein Verfahren beschrieben werden, um aus großen Datenmengen Informationen zu extrahieren und aus diesen Daten ein Modell zu erzeugen, welches es erlaubt, auch für Werte, die nicht Teil des Datensatzes sind, Vorhersagen zu treffen. Bei Anwendung eines Prädiktive Analytik Verfahrens kann typischerweise ein Teil des Datensatzes als Trainings-Datensatz (auch als Trainingssatz oder Trainingsdaten bezeichnet) genutzt werden. Anhand dieses Trainingsdatensatzes können ein oder mehrere Modelle erzeugt werden, welche dann anhand der Daten, die nicht Teil des Trainingsdatensatzes sind, anhand der gesamten Daten, oder anhand eines speziell ausgewählten Teils der Daten, getestet werden können.

Für eine Bewertung des Modells, also eine Ermittlung der Anpassungsgüte, können beispielsweise ein Bestimmtheitsmaß $R^2$, ein mittlerer absoluter Fehler, ein mittlerer quadratischer Fehler, eine Standardabweichung und/oder eine mittlere Abweichung herangezogen werden.

Das Bestimmtheitsmaß $R^2$ kann für ein lineares Regressionsmodell einem quadrierten Korrelationskoeffizienten entsprechen. Für ein anderes Modell (eine andere Beziehung) kann es anders definiert sein.

Für die Modellierung mittels Prädiktiver Analytik können gemäß verschiedenen Ausführungsbeispielen verschiedene Funktionen oder Verfahren herangezogen werden. In einem einfachen Fall kann beispielsweise eine multiple lineare Regression genutzt werden. Bessere Ergebnisse können typischerweise unter Verwendung von (multiplen) polynomen Regressionen, neuronalen Netzen, Support Vector Machines, Entscheidungsbäumen (beispielsweise Tree-Ensembles) oder ähnlichem erzielt werden.

In verschiedenen Ausführungsbeispielen kann ein Verfahren zum computergestützten Ermitteln eines Haarfärbemittels zum Färben von Haaren in einer Wunschhaarfarbe eine folgende Abfolge (einen so genannten Workflow) aufweisen:

1. Bestimmen einer Ausgangshaarfarbe eines Benutzers (z.B. eines Verbrauchers), z.B. an verschiedenen Stellen seiner Haare (z.B. der Frisur), z.B. an Haaransatz und Spitzen, insbesondere an einer vom Benutzer als repräsentativ eingeschätzten Haarpartie;

2. Ermitteln (z.B. klassifizieren) weiterer relevanter Ausgangszustandsparameter, z.B. einer Haarschädigung (eines Schädigungsgrads) und/oder eines Ergrauungsgrads;

3. Ermitteln eines Wunschresultats (der Wunschhaarfarbe) des Benutzers, beispielsweise als Wertekombination im L*a*b*-Farbraum;

4. Berechnen einer Mehrzahl von, z.B. auf der als repräsentativ eingeschätzten Haarpartie, mittels allen zur Verfügung stehenden (z.B. im Handel oder bei einem Friseur erhältlichen vorkonfektionierten Färbemitteln (Retail-Produkten) erzielbaren Haarfarben;

5. Ermitteln (z.B. berechnen) eines Farbabstands $\Delta E$ für alle Kombinationen von erzielbarer Haarfarbe und Wunschhaarfarbe; und

6. Auswählen des vorkonfektionierten Färbemittels mit dem geringsten Farbabstand $\Delta E$.

In verschiedenen umfassenderen alternativen Ausführungsbeispielen kann ein Verfahren zum computergestützten Ermitteln eines Haarfärbemittels zum Färben von Haaren in einer Wunschhaarfarbe sich bei dem Haarfärbemittel nicht nur auf ein existierendes Portfolio von (vorkonfektionierten) Haarfärbemitteln (bzw. deren Rezepturen) beschränken, sondern alle in einem vorgegebenen n-dimensionalen Farbstoffraum (mit n Farbstoffvorstufen) denkbaren Rezepturen durch umfassende Permutation aller theoretischen Farbstoffvorstufenkombinationen einbeziehen und ein Farbresultat (z.B. eine Mehrzahl von erzielbaren Haarfarben) auf einer Ausgangslage (z.B. einer Mehrzahl von Ausgangsparametern) des Benutzers berechnen. Das Verfahren kann eine folgende Abfolge aufweisen:

1. Bestimmen einer Ausgangshaarfarbe eines Benutzers (z.B. eines Verbrauchers), z.B. an verschiedenen Stellen seiner Haare (z.B. der Frisur), z.B. an Haaransatz und Spitzen, insbesondere an einer vom Benutzer als repräsentativ eingeschätzten Haarpartie;

2. Ermitteln (z.B. klassifizieren) weiterer relevanter Ausgangszustandsparameter, z.B. einer Haarschädigung (eines Schädigungsgrads) und/oder eines Ergrauungsgrads;

3. Ermitteln eines Wunschresultats (der Wunschhaarfarbe) des Benutzers, beispielsweise als Wertekombination im L*a*b*-Farbraum;

4. Identifizieren von hochrelevanten und weniger relevanten Farbstoffen (z.B. Farbstoffvorstufen) in einem Predictive-Analytics-Modell (dies kann auch als "Feature Selection" bezeichnet werden);

5. Simulieren einer großen Zahl (z.B. >> $10^5$) theoretischer Rezepturen auf der repräsentativen Haarfarbe (einer Grundhaarfarbe der als repräsentativ eingeschätzten Haarpartie) des Benutzers;

6. Ermitteln (z.B. berechnen) eines Farbabstands $\Delta E$ für alle Kombinationen von erzielbarer Haarfarbe und Wunschhaarfarbe; und

7. Auswählen der theoretischen Rezeptur mit dem geringsten Farbabstand $\Delta E$ als das Haarfärbemittel;

8. Fertigen genau dieser Rezeptur, beispielsweise auf speziellen Produktionslinien oder direkt am Verkaufsort (auch als Point of Sale bezeichnet).

Alternativ zum Farbstand $\Delta E$ können in dem Verfahren auch die Farbtonabweichung $\Delta H$, die Helligkeitsabweichung $\Delta L$ oder die Chromaabweichung $\Delta C$ berücksichtigt werden.

Dieses Ausführungsbeispiel kann eine große Bedeutung haben für eine Realisierung einer individualisierte- Massenfertigung-Strategie ("individualisierte Haarfarben", auch als Mass Customization Strategie bezeichnet) im Bereich Haarfaben.

In verschiedenen Ausführungsbeispielen wird ein Verfahren zum computergestützten Ermitteln eines Haarfärbemittels zum Färben von Haaren in einer Wunschhaarfarbe bereitgestellt. Das Verfahren kann aufweisen: Ermitteln eines Werts bzw. von Werten für mindestens einen Ausgangszustandsparameter, welcher einen Ausgangszustand der zu färbenden Haare beschreibt, Ermitteln einer Mehrzahl von mittels Färbens mit jeweils einem verfügbaren Haarfärbemittel erzielbaren Haarfarben, basierend auf einer anhand von Haarfarben-Daten für eine Mehrzahl von Färbevorgängen ermittelten Beziehung zwischen einer Mehrzahl von Färbevoraussetzungsparametern und erzielten Haarfarben, wobei die Mehrzahl von Färbevoraussetzungsparametern den jeweiligen Färbevorgang beeinflusst, und die erzielten Haarfarben mittels des jeweiligen Färbevorgangs erzielt werden, und dem Wert bzw. den Werten für den mindestens einen Ausgangszustandsparameter, Auswählen der Wunschhaarfarbe aus der Mehrzahl von mittels Färbens mit jeweils einem verfügbaren Haarfärbemittel erzielbaren Haarfarben, und Bestimmen des Haarfärbemittels zum Färben von Haaren in der Wunschhaarfarbe basierend auf der Wunschhaarfarbe und der ermittelten Beziehung zwischen den Färbevoraussetzungsparametern und den erzielten Haarfarben.

Gemäß verschiedenen Ausführungsformen kann das Bestimmen des Haarfärbemittels zum Färben von Haaren in der Wunschhaarfarbe aufweisen: Ermitteln einer Mehrzahl von Farbabständen, wobei jeder Farbabstand von der Mehrzahl von Farbabständen ein Farbabstand sein kann zwischen der Wunschhaarfarbe und einer anhand der Beziehung errechneten Haarfarbe für jeweils eines von einer Mehrzahl von vorkonfektionierten Haarfärbemitteln, Ermitteln eines minimalen Farbabstands aus der Mehrzahl von Farbabständen und Ermitteln des dem minimalen Farbabstand zugeordneten vorkonfektionierten Haarfärbemittels als das Haarfärbemittel zum Färben von Haaren in der Wunschhaarfarbe.

Gemäß verschiedenen Ausführungsformen kann das Bestimmen des Haarfärbemittels zum Färben von Haaren in der Wunschhaarfarbe aufweisen: Ermitteln, ausgehend von der Wunschhaarfarbe und unter Verwendung der ermittelten Beziehung zwischen der Mehrzahl von Färbevoraussetzungsparametern und den erzielten Haarfarben, einer Kombination von Farbstoffvorstufenkonzentrationen, welche gemäß der Beziehung bei einem Färben des Haars die Wunschhaarfarbe ergibt, wobei die Kombination von Farbstoffvorstufenkonzentrationen Bestandteil des Haarfärbemittels zum Färben von Haaren in der Wunschhaarfarbe sein kann.

Gemäß verschiedenen Ausführungsformen kann der mindestens eine Ausgangszustandsparameter eine Grundhaarfarbe aufweisen.

Gemäß verschiedenen Ausführungsformen kann der mindestens eine Ausgangszustandsparameter ferner einen Ergrauungsgrad oder/und eine Vorschädigung des Haars aufweisen.

Gemäß verschiedenen Ausführungsformen kann das Verfahren ferner aufweisen: Bereitstellen eines Toleranzbereichs, wobei der Toleranzbereich ein Farbabstand zur Wunschhaarfarbe sein kann, der toleriert wird, wobei das Bestimmen des Haarfärbemittels zum Färben von Haaren in der Wunschhaarfarbe zusätzlich auf dem Toleranzbereich basieren kann.

In all diesen Ausführungsformen kann der Farbabstand $\Delta E$ mit der Farbtonabweichung $\Delta H$ und/oder der

Helligkeitsabweichung ΔL gewichtet werden.

Gemäß verschiedenen Ausführungsformen kann der Toleranzbereich kleiner sein als eine menschliche Wahrnehmungsschwelle für den Farbabstand.

Gemäß verschiedenen Ausführungsformen kann das Verfahren ferner aufweisen: Bereitstellen eines Wunschwerts für mindestens einen zusätzlichen Färbeergebnisparameter, Ermitteln von mindestens einer erzielbaren Haarfarbe, welche den Wunschwert für den mindestens einen zusätzlichen Färbeergebnisparameter aufweisen kann, aus der Mehrzahl von mittels Färbens mit jeweils einem verfügbaren Haarfärbemittel erzielbaren Haarfarben, wobei das Auswählen der Wunschhaarfarbe aus der Mehrzahl von mittels Färbens mit jeweils einem verfügbaren Haarfärbemittel erzielbaren Haarfarben aus der mindestens einer erzielbaren Haarfarbe, welche den Wunschwert für den mindestens einen zusätzlichen Färbeergebnisparameter aufweisen kann, erfolgen kann.

Gemäß verschiedenen Ausführungsformen kann der mindestens eine zusätzliche Färbeergebnisparameter eine Waschechtheit, eine Lichtechtheit und/oder eine Fähigkeit zur Grauabdeckung aufweisen.

In einer vorteilhaften Ausgestaltung des Verfahrens werden die Farbstoffvorstufenkonzentrationen und/oder Farbstoffkonzentrationen in den Haarfärbemitteln nacheinander wie folgt vorhergesagt:

Zur Vorhersage einer ersten Farbstoffvorstufenkonzentration und/oder Farbstoffkonzentration c1 werden die sechs Farbeigenschaften des LAB-Farbraums der Ausgangshaarfarbe (L1, a1 und b1) und der Wunschhaarfarbe (L2, a2 und b2) verwendet. Zur Vorhersage einer zweiten Farbstoffvorstufenkonzentration und/oder Farbstoffkonzentration c2 werden zusätzlich zu den sechs oben genannten Farbeigenschaften die zuvor vorhergesagte Farbstoffvorstufenkonzentration und/oder Farbstoffkonzentration c1 als weitere Eigenschaft verwendet. Entsprechend werden bei der Vorhersage einer Farbstoffvorstufenkonzentration und/oder Farbstoffkonzentration c3 neben den sechs oben genannten Farbeigenschaften die zuvor vorhergesagten Farbstoffvorstufenkonzentration und/oder Farbstoffkonzentration c1 und c2 verwendet, usw.

In einer weiteren vorteilhaften Ausgestaltung des Verfahrens erfolgt die Modellierung mittels Prädiktiver Analytik unter Verwendung eines Tree-Ensemble-Modells.

In verschiedenen Ausführungsbeispielen wird eine Datenverarbeitungsvorrichtung zum Ausführen eines computergestützten Ermittelns eines Haarfärbemittels zum Färben von Haaren in einer Wunschhaarfarbe bereitgestellt, wobei die Datenverarbeitungsvorrichtung eingerichtet sein kann, das Verfahren gemäß Anspruch 1 auszuführen.

In verschiedenen Ausführungsbeispielen wird eine Vorrichtung zum Herstellen eines individuell ermittelten Haarfärbemittels zum Färben von Haaren in einer Wunschhaarfarbe bereitgestellt, aufweisend: eine Datenverarbeitungsvorrichtung gemäß Anspruch 10 und eine Haarfärbemittel-Anfertigungsvorrichtung, aufweisend eine Mehrzahl von Farbstoffvorstufen, und eine Dosiervorrichtung zum Dosieren der Mehrzahl von Farbstoffvorstufen, wobei eine Kombination von Farbstoffvorstufenkonzentrationen des Haarfärbemittels mittels eines Verfahrens gemäß Anspruch 3 ermittelt werden kann, und wobei ein Dosieren der Mehrzahl von Farbstoffvorstufen mittels der Dosiervorrichtung anhand der Kombination von Farbstoffvorstufenkonzentrationen und des Haarfärbemittels erfolgen kann.

In verschiedenen Ausführungsformen kann die Vorrichtung an einem Verkaufsort für Haarbehandlungsprodukte bereitgestellt sein.

Ausführungsbeispiele der Offenbarung sind in den Figuren dargestellt und werden im Folgenden näher erläutert.

Es zeigen

Figur 1     Haarfarben-Daten gemäß einem Ausführungsbeispiel;

Figur 2     eine schematische Darstellung eines Verfahrens zum computergestützten Ermitteln eines Haarfärbemittels zum Färben von Haaren in einer Wunschhaarfarbe;

Figur 3     ein Ablaufdiagramm, welches ein Verfahren zum computergestützten Ermitteln eines Haarfärbemittels zum Färben von Haaren in einer Wunschhaarfarbe gemäß verschiedenen Ausführungsbeispielen darstellt;

Figur 4     eine schematische Darstellung einer Datenverarbeitungsvorrichtung gemäß verschiedenen Ausführungsbeispielen; und

Figur 5     eine schematische Darstellung einer Vorrichtung zum Herstellen eines individuell ermittelten Haarfärbemittels zum Färben von Haaren in einer Wunschhaarfarbe gemäß verschiedenen Ausführungsbeispielen.

In der folgenden ausführlichen Beschreibung wird auf die beigefügten Zeichnungen Bezug genommen, die Teil der vorliegenden Anmeldung bilden und in denen zur Veranschaulichung spezifische Ausführungsformen gezeigt sind, in denen die Erfindung ausgeübt werden kann. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

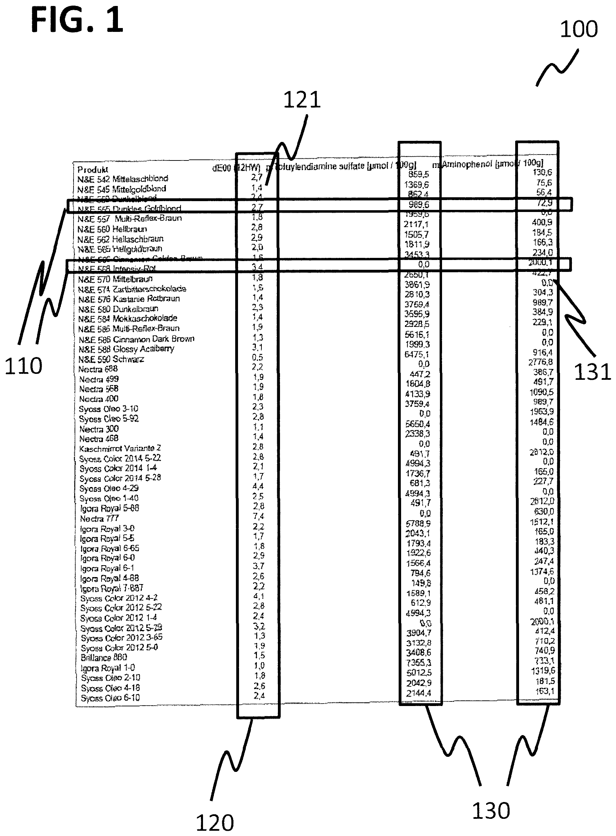

Figur 1 zeigt Haarfarben-Daten 100 gemäß einem Ausführungsbeispiel.

Die Haarfarben-Daten 100 weisen für eine Mehrzahl von Färbevorgängen (davon sind zwei beispielhaft mit 110 gekennzeichnet) eine Mehrzahl von Färbevoraussetzungsparametern 130 (Konzentrationen von jeweils einer Farbstoffvorstufe) und einen Färbergebnisparameter 120 (einen Farbabstand ΔE nach 12 Haarwäschen) auf. Ein beispielhafter Wert für einen der Färbevoraussetzungsparameter ist mit 131 gekennzeichnet. Ein beispielhafter Wert für einen Färbergebnisparameter ist mit 121 gekennzeichnet. Zusätzlich zu den hier dargestellten Parametern der Haarfarben-Daten 100 können die Haarfarben-Daten noch Werte für mindestens einen Ausgangszustandsparameter (nicht dargestellt, beispielsweise eine Grundhaarfarbe, einen Schädigungszustand, einen Ergrauungsgrad oder Ähnliches) aufweisen.

Die in den Tabellen wiedergegebenen Daten sind lediglich als veranschaulichende Beispiele zu verstehen. Welche Haarfarben-Daten in verschiedenen Ausführungsbeispielen für das Verfahren genutzt werden, hängt von einer konkreten Anwendung ab. Beispielsweise können andere, weniger oder mehr Produkte verwendet werden, welche ggf. andere, mehr oder weniger Farbstoffvorstufen oder zusätzlich (direktziehende) Farbstoffe aufweisen können, zusätzlich zur Farbe können weitere Parameter ermittelt und in den Haarfarben-Daten angegeben werden, wie beispielsweise eine Lichtechtheit, eine Grundhaarfarbe, usw.

Ein Bereitstellen der Haarfarben-Daten als Tabelle ist lediglich beispielhaft. Die Haarfarben-Daten können in jeder beliebigen Form bereitgestellt werden, welche ein Zuordnen der Färbeergebnisparameter zu den jeweiligen zugehörigen Färbevoraussetzungsparametern und ein computergestütztes Verwenden der Haarfarben-Daten ermöglicht.

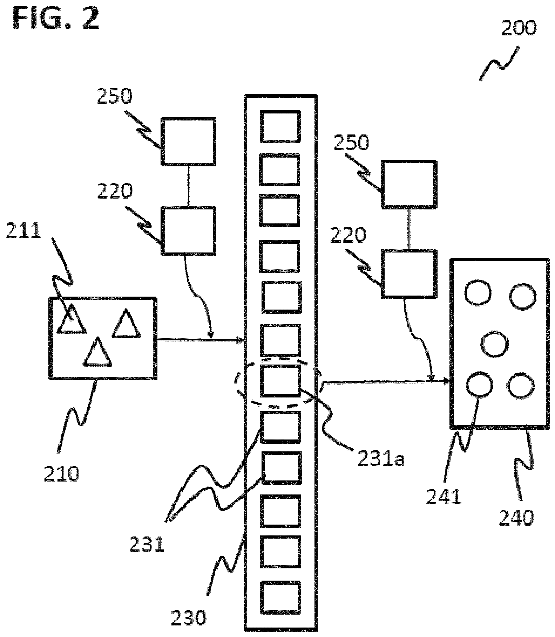

Figur 2 zeigt eine schematische Darstellung 200 eines Verfahrens zum computergestützten Ermitteln eines Haarfärbemittels zum Färben von Haaren in einer Wunschhaarfarbe.

In verschiedenen Ausführungsbeispielen kann ein Ausgangszustand 210 bereitgestellt sein. Der Ausgangszustand 210 kann einen Wert für mindestens einen Ausgangszustandsparameter 211, beispielsweise für eine Mehrzahl von Ausgangszustandsparametern 211, aufweisen.

Der mindestens eine Ausgangszustandsparameter 211 kann beispielsweise mindestens eine Ausgangshaarfarbe der zu färbenden Haare aufweisen, wobei die Ausgangshaarfarbe in einem Farbraum, beispielsweise einem L*a*b*-Farbraum, einem RGB-Farbraum, o.ä., parametrisiert sein kann.

In verschiedenen Ausführungsbeispielen kann die mindestens eine Ausgangshaarfarbe eine Mehrzahl von Ausgangshaarfarben aufweisen, beispielsweise eine erste Ausgangshaarfarbe an den Spitzen der Haare, einer zweite Ausgangshaarfarbe an einem Haaransatz der Haare und/oder eine dritte Ausgangshaarfarbe an einem Hauptteil der Haare, und/oder eine vierte Ausgangshaarfarbe an Strähnen der Haare, o.ä.

Eine für die Haare repräsentative Haarfarbe kann beispielsweise vom Benutzer bestimmt werden.

Die Ausgangshaarfarbe kann in verschiedenen Ausführungsbeispielen mittels Messens ermittelt werden, z.B. mittels einer farbmetrischen Vermessung, beispielsweise unter definierten Bedingungen, z.B. hinsichtlich Lichtart usw.

Der mindestens eine Ausgangszustandsparameter 211 kann zusätzlich zur Ausgangshaarfarbe einen Ergrauungsgrad der Haare, einen Schädigungsgrad der Haare o.ä. aufweisen.

In verschiedenen Ausführungsbeispielen kann eine Mehrzahl von mittels Färbens mit jeweils einem verfügbaren Haarfärbemittel von einer Mehrzahl von verfügbaren Haarfärbemitteln erzielbaren Haarfarben 231 ermittelt werden.

Die Mehrzahl von erzielbaren Haarfarben 231 kann eine Gesamtheit 230 von erzielbaren Haarfarben bilden.

Dem Ermitteln der erzielbaren Haarfarben können in verschiedenen Ausführungsbeispielen eine Beziehung 220 zwischen einer Mehrzahl von Färbevoraussetzungsparametern 250 und erzielten Haarfarben 231, welche basierend auf einer anhand von Haarfarben-Daten für eine Mehrzahl von Färbevorgängen ermittelt wurde oder wird, und der mindestens eine Wert für den mindestens einen Ausgangszustandsparameter 211 zugrunde gelegt werden.

In verschiedenen Ausführungsbeispielen kann beim Ermitteln der erzielbaren Haarfarben 231 mindestens ein Wunschwert für einen zusätzlichen (z.B. zusätzlich zur Haarfarbe) Färbeergebnisparameter berücksichtigt werden (nicht dargestellt). Beispielsweise können die erzielbaren Haarfarben beim Ermitteln der erzielbaren Haarfarben 231 auf diejenigen beschränkt werden, die auch den (z.B. Mindest-) Wert für den mindestens einen zusätzlichen Färbeergebnisparameter, beispielsweise eine geforderte (Mindest-)Waschechtheit oder eine geforderte (Mindest-)Grauabdeckung aufweist.

In verschiedenen Ausführungsbeispielen kann die Mehrzahl von verfügbaren Haarfärbemitteln eine Mehrzahl von vorkonfektionierten Haarfärbemitteln aufweisen. In dem Fall kann die Gesamtheit 230 von erzielbaren Haarfarben diskrete Werte für die erzielbaren Haarfarben 231 aufweisen.

In verschiedenen Ausführungsbeispielen kann die Mehrzahl von verfügbaren Haarfärbemitteln eine Mehrzahl von Haarfärbemitteln aufweisen, welche jeweils eine zuvor noch nicht bekannte, nicht verwendete oder nicht ausgefärbte Kombination von Konzentrationen von Farbstoffvorstufen aufweisen. In dem Fall kann die Gesamtheit 230 von erzielbaren Haarfarben eine kontinuierliche Verteilung von erzielbaren Haarfarben 231 aufweisen.

In verschiedenen Ausführungsbeispielen kann die Mehrzahl von verfügbaren Haarfärbemitteln sowohl vorkonfektionierte als auch unbekannte Haarfärbemittel aufweisen.

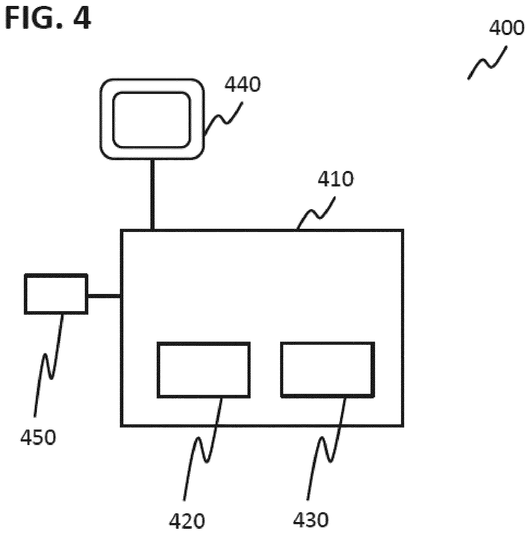

In verschiedenen Ausführungsbeispielen kann das Verfahren mittels einer Datenverarbeitungsvorrichtung (siehe Figur 4 und zugehörige Beschreibung) ausgeführt werden.

Aus den erzielbaren Haarfarben 231, die mittels eines jeweiligen Färbevorgangs mit einem Haarfärbemittel erzielt werden können, kann in verschiedenen Ausführungsbeispielen eine Wunschhaarfarbe 231a ausgewählt werden.

Das Auswählen der Wunschhaarfarbe 231a kann in verschiedenen Ausführungsbeispielen beispielsweise ein Auswählen nach einem Präsentieren der erzielbaren Haarfarben 231 aufweisen.

Unter Verwendung der Wunschhaarfarbe 231a, der Beziehung (des Modells) 220 und der Färbevoraussetzungsparameter 250 kann das Haarfärbemittel 240 zum Färben der Haare in der Wunschhaarfarbe 231a ermittelt werden. Dabei kann das Haarfärbemittel 240 eine Mehrzahl von Farbstoffvorstufen 241 aufweisen, deren Konzentrationen (und somit ein relatives Mengenverhältnis, z.B. ein Mischungsverhältnis) beim Ermitteln des Haarfärbemittels 240 bestimmt werden.

In verschiedenen Ausführungsbeispielen kann das Haarfärbemittel 240 dasjenige Haarfärbemittel 240 aus einer Mehrzahl von Haarfärbemitteln sein, welches zu einem minimalen Farbabstand zwischen der Wunschhaarfarbe und der mittels des Haarfärbemittels 240 erzielten Haarfarbe führt.

In verschiedenen Ausführungsbeispielen kann die prädiktive Analytik mittels der Software KNIME 2.11.2 durchgeführt werden. Alternativ kann jede andere zum Durchführen des Verfahrens geeignete Software verwendet werden.

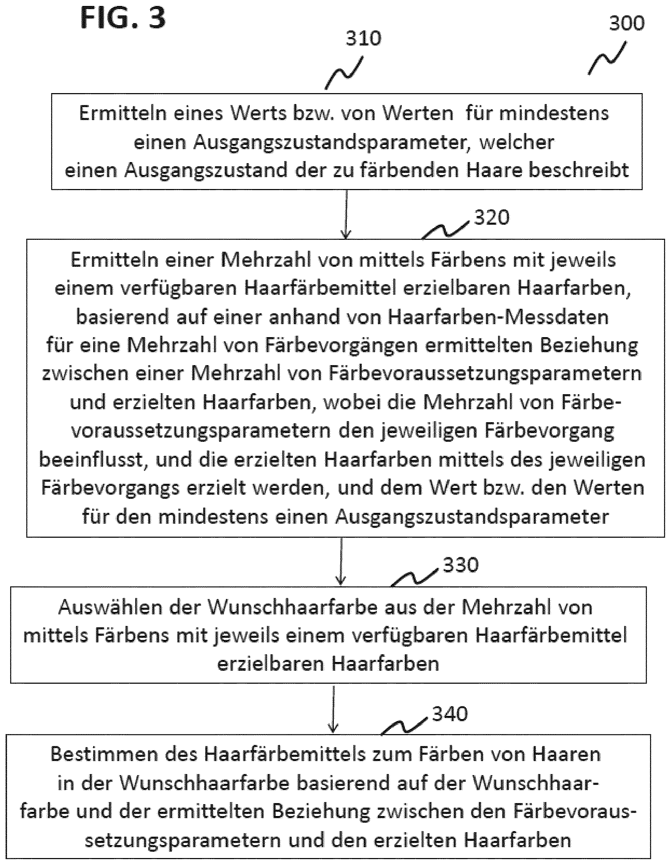

Figur 3 zeigt ein Ablaufdiagramm 300, welches ein Verfahren zum computergestützten Ermitteln eines Haarfärbemittels zum Färben von Haaren in einer Wunschhaarfarbe gemäß verschiedenen Ausführungsbeispielen darstellt.

In verschiedenen Ausführungsbeispielen kann das Verfahren aufweisen: Ermitteln eines Werts bzw. von Werten für mindestens einen Ausgangszustandsparameter, welcher einen Ausgangszustand der zu färbenden Haare beschreibt (bei 310); Ermitteln einer Mehrzahl von mittels Färbens mit jeweils einem verfügbaren Haarfärbemittel erzielbaren Haarfarben, basierend auf einer anhand von Haarfarben-Daten für eine Mehrzahl von Färbevorgängen ermittelten Beziehung zwischen einer Mehrzahl von Färbevoraussetzungsparametern und erzielten Haarfarben, wobei die Mehrzahl von Färbevoraussetzungsparametern den jeweiligen Färbevorgang beeinflusst, und die erzielten Haarfarben mittels des jeweiligen Färbevorgangs erzielt werden, und dem Wert bzw. den Werten für den mindestens einen Ausgangszustandsparameter (bei 320); Auswählen der Wunschhaarfarbe aus der Mehrzahl von mittels Färbens mit jeweils einem verfügbaren Haarfärbemittel erzielbaren Haarfarben (bei 330); und Bestimmen des Haarfärbemittels zum Färben von Haaren in der Wunschhaarfarbe basierend auf der Wunschhaarfarbe und der ermittelten Beziehung zwischen den Färbevoraussetzungsparametern und den erzielten Haarfarben (bei 340).

Figur 4 zeigt eine schematische Darstellung einer Datenverarbeitungsvorrichtung 400 zum Ausführen des Verfahrens zum Ermitteln eines Haarfärbemittels zum Färben von Haaren in einer Wunschhaarfarbe gemäß verschiedenen Ausführungsbeispielen.

Die Datenverarbeitungsvorrichtung 400 kann beispielsweise einen Computer aufweisen, oder jede andere Datenverarbeitungsvorrichtung, die geeignet ist, die Daten zu speichern und bereitzustellen, das Prädiktive-Analytik-Verfahren auszuführen und das Haarfärbemittel anhand der Wunschhaarfarbe zu ermitteln, also beispielsweise jede Datenverarbeitungsvorrichtung mit hinreichend großem Datenspeicher und hinreichend leistungsfähigem Prozessor.

Die Datenverarbeitungsvorrichtung 400 kann in verschiedenen Ausführungsbeispielen einen Prozessor 420 aufweisen. Der Prozessor 420 kann beispielsweise ein Mikroprozessor der Datenverarbeitungsvorrichtung 400 sein oder einen solchen Mikroprozessor aufweisen.

In verschiedenen Ausführungsbeispielen kann die Datenverarbeitungsvorrichtung 400 eine Datenspeichervorrichtung 430 aufweisen. Die Datenspeichervorrichtung kann ein interner oder externer Datenspeicher 430 einer der genannten Datenverarbeitungsvorrichtungen 400 sein oder einen solchen Datenspeicher 430 aufweisen. Der Datenspeicher 430 kann eingerichtet sein, Daten zu speichern, welche bei einer Durchführung des Verfahrens zum Ermitteln eines Haarfärbemittels zum Färben von Haaren in einer Wunschhaarfarbe gespeichert und/oder abgerufen werden, beispielsweise Haarfarben-Daten oder Ausgangszustandsparameter.

In verschiedenen Ausführungsbeispielen kann die Datenverarbeitungsvorrichtung 400 eine Anzeigevorrichtung 440 aufweisen. Die Anzeigevorrichtung 440 kann beispielsweise einen Bildschirm eines PCs, eines Laptops oder einer sonstigen beliebigen Datenverarbeitungsvorrichtung 400 aufweisen. Die Anzeigevorrichtung 440 kann beispielsweise genutzt werden, um Ergebnisse des Verfahrens zum Ermitteln eines Haarfärbemittels zum Färben von Haaren in einer Wunschhaarfarbe darzustellen, Eingabeparameter für das Ausführen des Verfahrens zu erfragen, oder ähnliches.

In verschiedenen Ausführungsbeispielen kann die Datenverarbeitungsvorrichtung 400 eine Eingabevorrichtung 450 zum Bereitstellen von Informationen an die Datenverarbeitungsvorrichtung 400 aufweisen, beispielsweise eine Tastatur, eine Maus, eine berührungsempfindliche Oberfläche der Anzeigevorrichtung 440, oder ähnliches.

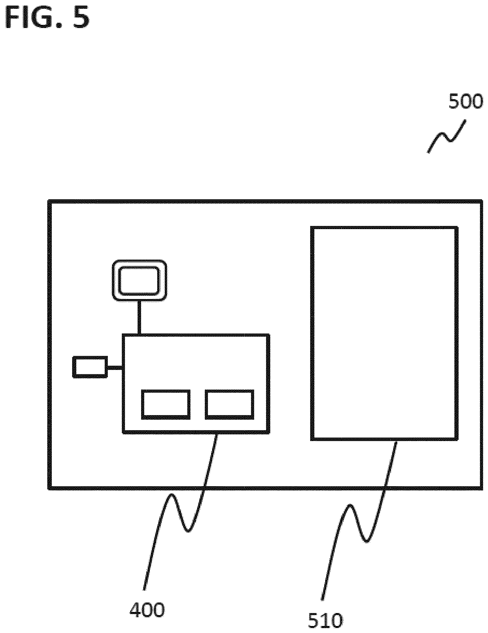

Figur 5 zeigt eine schematische Darstellung einer Vorrichtung 500 zum Herstellen eines individuell ermittelten Haarfärbemittels zum Färben von Haaren in einer Wunschhaarfarbe gemäß verschiedenen Ausführungsbeispielen.

Die Vorrichtung 500 zum Herstellen eines individuell ermittelten Haarfärbemittels zum Färben von Haaren in einer Wunschhaarfarbe kann eine Datenverarbeitungsvorrichtung 400 (siehe Fig.4 und zugehörige Beschreibung) aufweisen.

Die Vorrichtung 500 kann ferner eine Haarfärbemittel-Anfertigungsvorrichtung 510 aufweisen.

Der Haarfärbemittel-Anfertigungsvorrichtung 510 kann eine Mehrzahl von Farbstoffvorstufen und eine Dosiervorrichtung zum Dosieren der Mehrzahl von Farbstoffvorstufen (nicht dargestellt) bereitgestellt sein.

Zum Herstellen des Haarfärbemittels können die Farbstoffvorstufen anhand der ermittelten Farbstoffvorstufenkonzentrationen (siehe beispielsweise Fig.2 und zugehörige Beschreibung, z.B. dort die Farbstoffvorstufen 241) dosiert werden.

Eine Kombination von Farbstoffvorstufenkonzentrationen des Haarfärbemittels kann beispielsweise mittels eines Verfahrens wie in Fig.3 und zugehöriger Beschreibung dargestellt, ermittelt werden.

Die Haarfärbemittel-Anfertigungsvorrichtung 510 kann in verschiedenen Ausführungsbeispielen an einem Produktionsstandort für Haarfärbemittel bereitgestellt sein. Alternativ kann die Haarfärbemittel-Anfertigungsvorrichtung 510 an einem Verkaufs- und/oder Verwendungsort von Haarfärbemitteln bereitgestellt sein.

Das Bereitstellen der Haarfärbemittel-Anfertigungsvorrichtung 510 erlaubt ein sofortiges Herstellen und bereitstellen eines individuell angepassten Haarfärbemittels.

Weitere vorteilhafte Ausgestaltungen des Verfahrens ergeben sich aus der Beschreibung der Vorrichtung und umgekehrt.

**Patentansprüche**

1. Verfahren zum computergestützten Ermitteln und Herstellen eines Haarfärbemittels zum Färben von Haaren in einer Wunschhaarfarbe, aufweisend:

   • Ermitteln eines Werts bzw. von Werten für mindestens einen Ausgangszustandsparameter, welcher einen Ausgangszustand der zu färbenden Haare beschreibt;
   • Ermitteln einer Mehrzahl von mittels Färbens mit jeweils einem verfügbaren Haarfärbemittel erzielbaren Haarfarben, basierend auf

      ∘ einer anhand von Haarfarben-Daten für eine Mehrzahl von Färbevorgängen ermittelten Beziehung zwischen einer Mehrzahl von Färbevoraussetzungsparametern und erzielten Haarfarben, wobei die Mehrzahl von Färbevoraussetzungsparametern den jeweiligen Färbevorgang beeinflusst, und die erzielten Haarfarben mittels des jeweiligen Färbevorgangs erzielt werden, und

      ∘ dem Wert bzw. den Werten für den mindestens einen Ausgangszustandsparameter;

   • Auswählen der Wunschhaarfarbe aus der Mehrzahl von mittels Färbens mit jeweils einem verfügbaren Haarfärbemittel erzielbaren Haarfarben,
   • Bestimmen des Haarfärbemittels zum Färben von Haaren in der Wunschhaarfarbe basierend auf der Wunschhaarfarbe und der ermittelten Beziehung zwischen den Färbevoraussetzungsparametern und den erzielten Haarfarben,

   wobei der mindestens eine Ausgangszustandsparameter eine Grundhaarfarbe aufweist, wobei die Mehrzahl von Färbevoraussetzungsparametern eine Mehrzahl von Konzentrationen von Farbstoffvorstufen und mindestens einen der Ausgangszustandsparameter aufweist; und
   wobei das Bestimmen des Haarfärbemittels zum Färben von Haaren in der Wunschhaarfarbe aufweist:

      • Ermitteln einer Mehrzahl von Farbabständen, wobei jeder Farbabstand von der Mehrzahl von Farbabständen ein Farbabstand ist zwischen der Wunschhaarfarbe und einer anhand der Beziehung errechneten Haarfarbe für jeweils eines von einer Mehrzahl von Haarfärbemitteln;
      • Ermitteln eines minimalen Farbabstands aus der Mehrzahl von Farbabständen; und Ermitteln des dem minimalen Farbabstand zugeordneten Haarfärbemittels als das Haarfärbemittel zum Färben von Haaren in der Wunschhaarfarbe,

   wobei das Ermitteln des Haarfärbemittels zum Färben von Haaren in der Wunschhaarfarbe aufweist:

      • Ermitteln, ausgehend von der Wunschhaarfarbe und unter Verwendung der ermittelten Beziehung zwischen der Mehrzahl von Färbevoraussetzungsparametern und den erzielten Haarfarben, einer Kombination von Farbstoffvorstufenkonzentrationen, welche gemäß der Beziehung bei einem Färben des Haars die Wunschhaarfarbe ergibt, wobei die Kombination von Farbstoffvorstufenkonzentrationen Bestandteil des Haarfärbemittels zum Färben von Haaren in der Wunschhaarfarbe ist,
      • Herstellen des ermittelten des Haarfärbemittels zum Färben von Haaren in der Wunschhaarfarbe.

2. Verfahren gemäß einem der vorherigen Ansprüche, wobei der mindestens eine Ausgangszustandsparameter ferner einen Ergrauungsgrad oder/und eine Vorschädigung des Haars aufweist.

3. Verfahren gemäß einem der vorherigen Ansprüche, ferner aufweisend:

   • Bereitstellen eines Toleranzbereichs, wobei der Toleranzbereich ein Farbabstand zur Wunschhaarfarbe ist, der toleriert wird,
   • wobei das Bestimmen des Haarfärbemittels zum Färben von Haaren in der Wunschhaarfarbe zusätzlich auf dem Toleranzbereich basiert.

4. Verfahren gemäß Anspruch 3,
   wobei der Toleranzbereich kleiner ist als eine menschliche Wahrnehmungsschwelle für den Farbabstand.

5. Verfahren gemäß einem der vorherigen Ansprüche, ferner aufweisend:

   • Bereitstellen eines Wunschwerts für mindestens einen zusätzlichen Färbeergebnisparameter; und
   • Ermitteln von mindestens einer erzielbaren Haarfarbe, welche den Wunschwert für den mindestens einen zusätzlichen Färbeergebnisparameter aufweist, aus der Mehrzahl von mittels Färbens mit jeweils einem verfügbaren Haarfärbemittel erzielbaren Haarfarben,

   wobei das Auswählen der Wunschhaarfarbe aus der Mehrzahl von mittels Färbens mit jeweils einem verfügbaren Haarfärbemittel erzielbaren Haarfarben aus der mindestens einer erzielbaren Haarfarbe, welche den Wunschwert für den mindestens einen zusätzlichen Färbeergebnisparameter aufweist, erfolgt,
   wobei der mindestens eine zusätzliche Färbeergebnisparameter eine Waschechtheit, eine Lichtechtheit und/oder eine Fähigkeit zur Grauabdeckung aufweist.

6. Vorrichtung zum Herstellen eines individuell ermittelten Haarfärbemittels zum Färben von Haaren in einer Wunschhaarfarbe, aufweisend:

   • eine Datenverarbeitungsvorrichtung zum Ausführen eines computergestützten Ermittelns eines Haarfärbemittels zum Färben von Haaren in einer Wunschhaarfarbe, wobei die Datenverarbeitungsvorrichtung eingerichtet ist, die computergestützten Schritte des Verfahrens gemäß Anspruch 1 auszuführen; und
   • eine Haarfärbemittel-Anfertigungsvorrichtung, aufweisend

      ∘ eine Mehrzahl von Farbstoffvorstufen; und
      ∘ eine Dosiervorrichtung zum Dosieren der Mehrzahl von Farbstoffvorstufen,

   • wobei eine Kombination von Farbstoffvorstufenkonzentrationen des Haarfärbemittels mittels eines Verfahrens gemäß Anspruch 1 ermittelt wird, und
   • wobei ein Dosieren der Mehrzahl von Farbstoffvorstufen mittels der Dosiervorrichtung anhand der Kombination von Farbstoffvorstufenkonzentrationen des Haarfärbemittels erfolgt.

7. Vorrichtung gemäß Anspruch 6, wobei die Vorrichtung an einem Verkaufsort für Haarbehandlungsprodukte bereitgestellt ist.

**Claims**

1. A method for determining and producing, in a computer-assisted manner, a hair dye for dyeing hair in a desired hair color, comprising:

   • determining a value or values for at least one initial condition parameter which describes an initial condition of the hair to be dyed;
   • determining a plurality of hair colors which can be achieved by dyeing using an available hair dye, based on

      ∘ a relationship, determined on the basis of hair color data for a plurality of dyeing processes, between a plurality of dyeing prerequisite parameters and achieved hair colors, wherein the plurality of dyeing prerequisite parameters influences the particular dyeing process, and the achieved hair colors are achieved by means of the particular dyeing process, and
      ∘ the value or values for the at least one initial condition parameter;

   • selecting the desired hair color from the plurality of hair colors which can be achieved by dyeing using an available hair dye;
   • determining the hair dye for dyeing hair in the desired hair color based on the desired hair color and the determined relationship between the dyeing prerequisite parameters and the achieved hair colors,

   wherein the at least one initial condition parameter has a basic hair color, wherein the plurality of dyeing prerequisite parameters comprises a plurality of concentrations of dye precursors and at least one of the initial condition parameters; and
   wherein determining the hair dye for dyeing hair in the desired hair color comprises:

• determining a plurality of color distances, wherein each color distance from the plurality of color distances is a color distance between the desired hair color and a hair color, calculated on the basis of the relationship, for one of a plurality of hair dyes;

• determining a minimum color distance from the plurality of color distances; and determining the hair dye associated with the minimum color distance as the hair dye for dyeing hair in the desired hair color, wherein the determination of the hair dye for dyeing hair in the desired hair color comprises:

• determining, proceeding from the desired hair color and using the determined relationship between the plurality of dyeing prerequisite parameters and the achieved hair colors, a combination of dye precursor concentrations which, according to the

relationship, results in the desired hair color when dyeing the hair, wherein the combination of dye precursor concentrations is part of the hair dye for dyeing hair in the desired hair color,

• producing the determined hair dye for dyeing hair in the desired hair color.

2. The method according to one of the preceding claims, wherein the at least one initial condition parameter further comprises a degree of graying and/or pre-damage to the hair.

3. The method according to one of the preceding claims, further comprising:

• providing a tolerance range, wherein the tolerance range is a tolerated color distance from the desired hair color,
• wherein the determination of the hair dye for dyeing hair in the desired hair color is additionally based on the tolerance range.

4. The method according to claim 3, wherein the tolerance range is less than a human perception threshold for the color distance.

5. The method according to one of the preceding claims, further comprising:

• providing a desired value for at least one additional dyeing result parameter; and
• determining at least one achievable hair color, which has the desired value for the at least one additional dyeing result parameter, from the plurality of hair colors which can be achieved by dyeing using an available hair dye,

wherein the desired hair color is selected from the plurality of hair colors that can be achieved by dyeing using an available hair dye from the at least one achievable hair color that has the desired value for the at least one additional dyeing result parameter, wherein the at least one additional dyeing result parameter has a wash fastness, a light fastness and/or a gray coverage ability.

6. A device for producing an individually determined hair dye for dyeing hair in a desired hair color, comprising:

• a data processing device for determining, in a computer-assisted manner, a hair dye for dyeing hair in a desired hair color, wherein the data processing device is designed to carry out the computer-assisted steps of the method according to claim 1; and
• a hair dye preparation device, comprising

◦ a plurality of dye precursors; and
◦ a dosing device for dosing the plurality of dye precursors,

• wherein a combination of dye precursor concentrations of the hair dye is determined by means of a method according to claim 1, and
• wherein the plurality of dye precursors is dosed by means of the dosing device on the basis of the combination of dye precursor concentrations of the hair dye.

7. The device according to claim 6, wherein the device is provided at a point of sale for hair treatment products.

**Revendications**

1. Procédé de détermination et de production assistées par ordinateur d'un agent de coloration capillaire permettant de colorer des cheveux dans une couleur de cheveux souhaitée, comprenant :

• la détermination d'une valeur ou de valeurs pour au moins un paramètre d'état initial qui décrit un état initial des cheveux à colorer ;
• la détermination d'une pluralité de couleurs de cheveux pouvant être obtenues au moyen de la coloration avec respectivement un agent de coloration capillaire disponible, sur la base

◦ d'une relation, déterminée à partir de données de couleurs de cheveux pour une pluralité de procédures de coloration, entre une pluralité de paramètres de condition de coloration et de couleurs de cheveux obtenues, la pluralité de paramètres de condition de coloration influençant la procédure de coloration respective, et les couleurs de cheveux obtenues étant obtenues au moyen de la procédure de coloration respective, et

◦ de la valeur ou des valeurs pour l'au moins un paramètre d'état initial ;

• la sélection de la couleur de cheveux souhaitée parmi la pluralité de couleurs de cheveux pouvant être obtenues au moyen de la coloration avec respectivement un agent de coloration capillaire disponible,
• la définition de l'agent de coloration capillaire permettant de colorer des cheveux dans la couleur de cheveux souhaitée sur la base de la couleur de cheveux souhaitée et de la relation déterminée entre les paramètres de condition de coloration et les couleurs de cheveux obtenues,

l'au moins un paramètre d'état initial comprenant une couleur de cheveux de base, la pluralité de paramètres de condition de coloration comprenant une pluralité de concentrations de précurseurs de couleur et au moins l'un des paramètres d'état initial ; et la définition de l'agent de coloration capillaire permettant de colorer des cheveux dans la couleur de cheveux souhaitée comprenant :

• la détermination d'une pluralité d'écarts de couleur, chaque écart de couleur de la pluralité d'écarts de couleur étant un écart de couleur entre la couleur de cheveux souhaitée et une couleur de cheveux calculée à partir de la relation pour respectivement un agent de coloration capillaire d'une pluralité d'agents de coloration capillaire ;
• la détermination d'un écart de couleur minimal parmi la pluralité d'écarts de couleur ; et

la détermination, comme agent de coloration capillaire, de l'agent de coloration capillaire associé à l'écart de couleur minimal permettant de colorer des cheveux dans la couleur de cheveux souhaitée, la détermination de l'agent de coloration capillaire permettant de colorer des cheveux dans la couleur de cheveux souhaitée comprenant :

• la détermination, en fonction de la couleur de cheveux souhaitée et en utilisant la relation déterminée entre la pluralité de paramètres de condition de coloration et les couleurs de cheveux obtenues, d'une combinaison de concentrations de précurseurs de couleur, laquelle permet d'obtenir la couleur de cheveux souhaitée selon la
relation lors d'une coloration des cheveux, la combinaison de concentrations de précurseurs de couleur faisant partie de l'agent de coloration capillaire permettant de colorer des cheveux dans la couleur de cheveux souhaitée,
• la production de l'agent de coloration capillaire déterminé permettant de colorer des cheveux dans la couleur de cheveux souhaitée.

2. Procédé selon l'une des revendications précédentes, dans lequel l'au moins un paramètre d'état initial comprend en outre un degré de grisonnement et/ou un endommagement préalable des cheveux.

3. Procédé selon l'une des revendications précédentes,
comprenant en outre :

• la mise à disposition d'une plage de tolérance, la plage de tolérance étant un écart de couleur toléré par rapport à la couleur de cheveux souhaitée,
• la définition de l'agent de coloration capillaire permettant de colorer des cheveux dans la couleur de cheveux souhaitée étant en outre basée sur la plage de tolérance.

4. Procédé selon la revendication 3, dans lequel la plage de tolérance est inférieure à un seuil de perception humaine pour l'écart de couleur.

5. Procédé selon l'une des revendications précédentes,
comprenant en outre :

• la mise à disposition d'une valeur souhaitée pour au moins un paramètre supplémentaire de résultat de coloration ; et
• la détermination d'au moins une couleur de cheveux pouvant être obtenue, laquelle comprend la valeur souhaitée pour l'au moins un paramètre supplémentaire de résultat de coloration, parmi la pluralité de couleurs de cheveux pouvant être obtenues au moyen de la coloration avec respectivement un agent de coloration capillaire disponible,

la sélection de la couleur de cheveux souhaitée parmi la pluralité de couleurs de cheveux pouvant être obtenues au moyen de la coloration avec respectivement un agent de coloration capillaire disponible étant effectuée parmi l'au moins une couleur de cheveux pouvant être obtenue, laquelle comprend la valeur souhaitée pour l'au moins un paramètre supplémentaire de résultat de coloration, l'au moins un paramètre supplémentaire de résultat de coloration comprenant une résistance au lavage, une résistance à la lumière et/ou une capacité de couverture des cheveux blancs.

6. Dispositif de production d'un agent de coloration capillaire déterminé individuellement permettant de colorer des cheveux dans une couleur de cheveux souhaitée, comprenant :

   • un dispositif de traitement de données permettant d'effectuer une détermination assistée par ordinateur d'un agent de coloration capillaire permettant de colorer des cheveux dans une couleur de cheveux souhaitée, le dispositif de traitement de données étant configuré pour mettre en œuvre les étapes du procédé assistées par ordinateur selon la revendication 1 ; et
   • un dispositif de préparation d'agent de coloration capillaire, comprenant

      ◦ une pluralité de précurseurs de couleur ; et
      ◦ un dispositif de dosage permettant de doser la pluralité de précurseurs de couleur,

   • une combinaison de concentrations de précurseurs de couleur de l'agent de coloration capillaire étant déterminée au moyen d'un procédé selon la revendication 1, et
   • un dosage de la pluralité de précurseurs de couleur étant effectué au moyen du dispositif de dosage à partir de la combinaison de concentrations de précurseurs de couleur de l'agent de coloration capillaire.

7. Dispositif selon la revendication 6, le dispositif étant mis à disposition à un point de vente pour des produits de traitement capillaire.

# FIG. 1

100

121

110

120

130

131

| Produkt | dE00 (12HW) | p-Toluylendiamine sulfate [µmol / 100g] | m-Aminophenol [µmol / 100g] |
|---|---|---|---|
| N&E 542 Mittelaschblond | 2,7 | 859,5 | 130,6 |
| N&E 545 Mittelgoldblond | 1,4 | 1369,6 | 75,6 |
| N&E 550 Dunkelblond | 2,4 | 852,4 | 56,4 |
| N&E 555 Dunkles Goldblond | 2,7 | 989,6 | 72,9 |
| N&E 557 Multi-Reflex-Braun | 1,8 | 1959,6 | 0,0 |
| N&E 560 Hellbraun | 2,8 | 2117,1 | 400,9 |
| N&E 562 Hellaschbraun | 2,9 | 1505,7 | 184,5 |
| N&E 565 Hellgoldbraun | 2,0 | 1811,9 | 166,3 |
| N&E 566 Cinnamon Goldes Braun | 1,6 | 3453,3 | 234,0 |
| N&E 568 Intensiv-Rot | 3,4 | 0,0 | 2000,1 |
| N&E 570 Mittelbraun | 1,8 | 2650,1 | 422,7 |
| N&E 574 Zartbitterschokolade | 1,5 | 3861,9 | 0,0 |
| N&E 576 Kastanie Rotbraun | 1,4 | 2810,3 | 304,3 |
| N&E 580 Dunkelbraun | 2,3 | 3759,4 | 989,7 |
| N&E 584 Mokkaschokolade | 1,4 | 3595,9 | 384,9 |
| N&E 586 Multi-Reflex-Braun | 1,9 | 2928,5 | 229,1 |
| N&E 586 Cinnamon Dark Brown | 1,3 | 5616,1 | 0,0 |
| N&E 588 Glossy Acaiberry | 3,1 | 1999,3 | 0,0 |
| N&E 590 Schwarz | 0,5 | 6475,1 | 916,4 |
| Nectra 688 | 2,2 | 0,0 | 2776,8 |
| Nectra 499 | 1,9 | 447,2 | 386,7 |
| Nectra 568 | 1,9 | 1604,8 | 491,7 |
| Nectra 400 | 1,8 | 4133,9 | 1090,5 |
| Syoss Oleo 3-10 | 2,3 | 3759,4 | 989,7 |
| Syoss Oleo 5-92 | 2,8 | 0,0 | 1963,9 |
| Nectra 300 | 1,1 | 5650,4 | 1484,6 |
| Nectra 468 | 1,4 | 2338,3 | 0,0 |
| Kaschmirrot Variante 2 | 2,8 | 0,0 | 2812,0 |
| Syoss Color 2014 5-22 | 2,8 | 491,7 | 0,0 |
| Syoss Color 2014 1-4 | 2,1 | 4994,3 | 165,0 |
| Syoss Color 2014 5-28 | 1,7 | 1736,7 | 227,7 |
| Syoss Oleo 4-29 | 4,4 | 681,3 | 0,0 |
| Syoss Oleo 1-40 | 2,5 | 4994,3 | 2812,0 |
| Igora Royal 5-88 | 2,8 | 491,7 | 630,0 |
| Nectra 777 | 7,4 | 0,0 | 1512,1 |
| Igora Royal 3-0 | 2,2 | 5788,9 | 165,0 |
| Igora Royal 5-5 | 1,7 | 2043,1 | 183,3 |
| Igora Royal 6-65 | 1,8 | 1793,4 | 440,3 |
| Igora Royal 6-0 | 2,9 | 1922,6 | 247,4 |
| Igora Royal 6-1 | 3,7 | 1566,4 | 1374,6 |
| Igora Royal 4-88 | 2,6 | 794,6 | 0,0 |
| Igora Royal 7-887 | 2,2 | 149,8 | 458,2 |
| Syoss Color 2012 4-2 | 4,1 | 1589,1 | 481,1 |
| Syoss Color 2012 5-22 | 2,8 | 612,9 | 0,0 |
| Syoss Color 2012 1-4 | 2,4 | 4994,3 | 2000,1 |
| Syoss Color 2012 5-29 | 3,2 | 0,0 | 412,4 |
| Syoss Color 2012 3-65 | 1,3 | 3904,7 | 710,2 |
| Syoss Color 2012 5-0 | 1,9 | 3132,8 | 740,9 |
| Brillance 880 | 1,5 | 3408,6 | 733,1 |
| Igora Royal 1-0 | 1,0 | 7355,3 | 1319,6 |
| Syoss Oleo 2-10 | 1,8 | 5012,5 | 181,5 |
| Syoss Oleo 4-18 | 2,6 | 2042,9 | 163,1 |
| Syoss Oleo 6-10 | 2,4 | 2144,4 | |

# FIG. 2

200

250

250

220

211

220

210

231a

231

230

241

240

# FIG. 3

310

300

Ermitteln eines Werts bzw. von Werten für mindestens
einen Ausgangszustandsparameter, welcher
einen Ausgangszustand der zu färbenden Haare beschreibt

320

Ermitteln einer Mehrzahl von mittels Färbens mit jeweils
einem verfügbaren Haarfärbemittel erzielbaren Haarfarben,
basierend auf einer anhand von Haarfarben-Messdaten
für eine Mehrzahl von Färbevorgängen ermittelten Beziehung
zwischen einer Mehrzahl von Färbevoraussetzungsparametern
und erzielten Haarfarben, wobei die Mehrzahl von Färbevoraussetzungsparametern den jeweiligen Färbevorgang
beeinflusst, und die erzielten Haarfarben mittels des jeweiligen
Färbevorgangs erzielt werden, und dem Wert bzw. den Werten
für den mindestens einen Ausgangszustandsparameter

330

Auswählen der Wunschhaarfarbe aus der Mehrzahl von
mittels Färbens mit jeweils einem verfügbaren Haarfärbemittel
erzielbaren Haarfarben

340

Bestimmen des Haarfärbemittels zum Färben von Haaren
in der Wunschhaarfarbe basierend auf der Wunschhaarfarbe und der ermittelten Beziehung zwischen den Färbevoraussetzungsparametern und den erzielten Haarfarben

**FIG. 4**

**FIG. 5**

500

400

510

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2006033907 A1
- WO 2006090363 A1
- WO 2011024160 A1
- EP 1374720 A1
- WO 2012127429 A2
- US 2004000015 A1
- WO 0187245 A2